(19) Europäisches Patentamt
European Patent Office
Office européen des brevets

(11) Veröffentlichungsnummer: **0 656 352 A1**

(12) **EUROPÄISCHE PATENTANMELDUNG**

(21) Anmeldenummer: **94118383.2**

(22) Anmeldetag: **23.11.94**

(51) Int. Cl.[6]: **C07C 327/58**, C07C 255/64, C07C 327/44, A01N 37/52

(30) Priorität: **03.12.93 DE 4341259**

(43) Veröffentlichungstag der Anmeldung: **07.06.95 Patentblatt 95/23**

(84) Benannte Vertragsstaaten: **AT BE CH DE DK ES FR GB GR IE IT LI NL PT SE**

(71) Anmelder: **BASF Aktiengesellschaft**
**Carl-Bosch-Strasse 38**
**D-67063 Ludwigshafen (DE)**

(72) Erfinder: **Bayer, Herbert, Dr.**
**D 3.4**
**D-68159 Mannheim (DE)**
Erfinder: **Sauter, Hubert, Dr.**
**Neckarpromenade 20**
**D-68167 Mannheim (DE)**
Erfinder: **Benoit, Remy, Dr.**
**Holzmühlstrasse 6**
**D-67435 Neustadt (DE)**
Erfinder: **Müller, Ruth, Dr.**
**Im Breitholz 3**
**D-56626 Andernach (DE)**
Erfinder: **Ammermann, Eberhard, Dr.**
**Von-Gagern-Strasse 2**
**D-64646 Heppenheim (DE)**
Erfinder: **Lorenz, Gisela, Dr.**
**Erlenweg 13**
**D-67434 Neustadt (DE)**

(54) **Thioimidate als Mittel gegen tierische Schädlinge und Schadpilze.**

(57) Thioimidate der Formel I

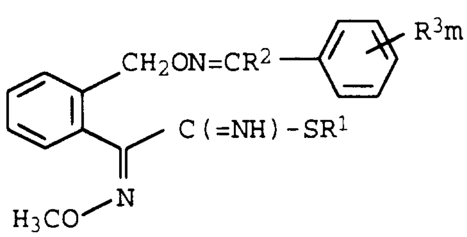

I

in der die Substituenten und der Index die folgende Bedeutung haben:

| | |
|---|---|
| $R^1$ | Alkyl; |
| $R^2$ | Alkyl oder Cyclopropyl; |
| m | 0, 1, 2 oder 3; |
| $R^3$ | Cyano, Nitro, Hydroxy, Mercapto, Halogen, Amino, ggf. subst. Alkyl, Cycloalkyl, ggf. subst. Alkoxy, Cycloalkoxy, Alkylthio, Alkylamino, Dialkylamino, Alkenyl, Alkenyloxy, Alkinyl, Alkinyloxy, Phenyl, Phenoxy, $C(=O)R^a$, $C(=O)OR^a$, $C(=O)NR^aR^b$, $C(=S)NR^aR^b$, $OC(=O)R^a$, $NR^aC(=O)R^b$, $NR^aC(=O)OR^b$, $S(=O)R^a$, $S(=O)OR^a$, $CR^a=NR^b$, $C(R^a)=NOR^b$, $C(XR^a)=NOR^b$, $N=CR^aR^b$, oder zwei benachbarte $R^3$ zusammen eine Gruppe CH=CH-CH=CH; |
| X | Sauerstoff, Schwefel oder Amino ($NR^c$); |
| $R^a$,$R^b$,$R^c$ | unabhängig voneinander Wasserstoff oder Alkyl, |

sowie deren Salze, Verfahren und Zwischenprodukte zu ihrer Herstellung und ihre Verwendung.

EP 0 656 352 A1

Die vorliegende Erfindung betrifft Thioimidate der Formel I

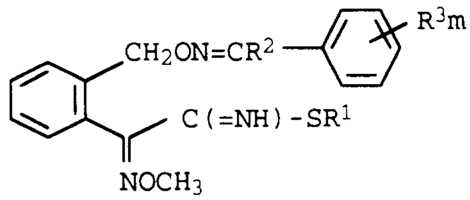

I

in der die Substituenten und der Index die folgende Bedeutung haben:

- $R^1$ $C_1$-$C_6$-Alkyl;
- $R^2$ $C_1$-$C_3$-Alkyl oder Cyclopropyl;
- m 0, 1, 2 oder 3, wobei die Reste $R^3$ verschieden sein können, wenn m für 2 oder 3 steht;
- $R^3$ Cyano, Nitro, Hydroxy, Mercapto, Halogen, Amino, $C_1$-$C_6$-Alkyl, Cyano-$C_1$-$C_6$-alkyl, Nitro-$C_1$-$C_6$-alkyl, $C_1$-$C_6$-Alkoxy-$C_1$-$C_6$-alkyl, $C_1$-$C_6$-Halogenalkyl, $C_6$-$C_6$-Cycloalkyl, $C_1$-$C_6$-Alkoxy, $C_1$-$C_6$-Halogenalkoxy, $C_3$-$C_6$-Cycloalkoxy, $C_1$-$C_6$-Alkylthio, $C_1$-$C_6$-Alkylamino, Di-$C_1$-$C_6$-alkylamino,
  $C_2$-$C_6$-Alkenyl, $C_2$-$C_6$-Alkenyloxy, $C_2$-$C_6$-Alkinyl, $C_2$-$C_6$-Alkinyloxy,
  Phenyl, Phenoxy,
  $C(=O)R^a$, $C(=O)OR^a$, $C(=O)NR^aR^b$, $C(=S)NR^aR^b$, $OC(=O)R^a$, $NR^aC(=O)R^b$, $NR^aC(=O)$-$OR^b$,$S(=O)R^a$, $S(=O)OR^a$,
  $CR^a=NR^b$, $C(R^a)=NOR^b$, $C(XR^a)=NOR^b$, $N=CR^aR^b$, oder zwei benachbarte $R^3$ zusammen eine Gruppe CH=CH-CH=CH;
- X Sauerstoff, Schwefel oder Amino ($NR^c$);
- $R^a$,$R^b$,$R^c$ unabhängig voneinander Wasserstoff oder $C_1$-$C_6$-Alkyl,

sowie deren Salze.

Außerdem betrifft die Erfindung Verfahren zur Herstellung dieser Verbindungen, insbesondere unter Verwendung der Verbindungen der Formel IX

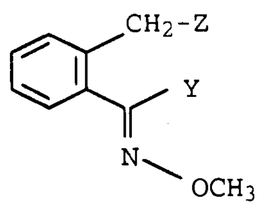

IX

in der die Substituenten die folgende Bedeutung haben:

- Y CN, $C(=S)NH_2$, $CO_2H$ oder $CONH_2$, und
- Z Wasserstoff, Halogen oder eine Gruppe

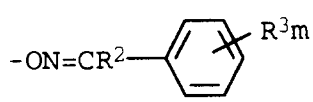

wobei $R^2$, $R^3$ und m die vorstehend gegebene Bedeutung haben, als Zwischenprodukte. Die Erfindung umfaßt desweiteren Mittel gegen tierische Schädlinge und Schadpilze, welche die Verbindungen I enthalten, die Verwendung der Verbindungen zur Herstellung solcher Mittel sowie ihre Verwendung zur Bekämpfung von tierischen Schädlingen oder Schadpilzen.

Aus der Literatur sind Thioimidate zur Schädlingsbekämpfung bekannt (EP-A 528 681).

Aufgabe der vorliegenden Erfindung waren neue Thioimidate mit verbessertem und verbreitertem Wirkungsspektrum.

Demgemäß wurden die eingangs definierten Thioimidate I gefunden. Außerdem wurden Verfahren und Zwischenprodukte zur Herstellung der Verbindungen I, sie enthaltende Mittel und ihre Verwendung zur

Bekämpfung tierischer Schädlinge oder Schadpilze gefunden. Die erfindungsgemäßen Verbindungen I sind auf verschiedenen Wegen zugänglich.

Besonders vorteilhaft erhält man sie auf dem im folgenden Reaktionsschema zusammengestellten Weg ausgehend von einem Toluolderivat der Formel II.

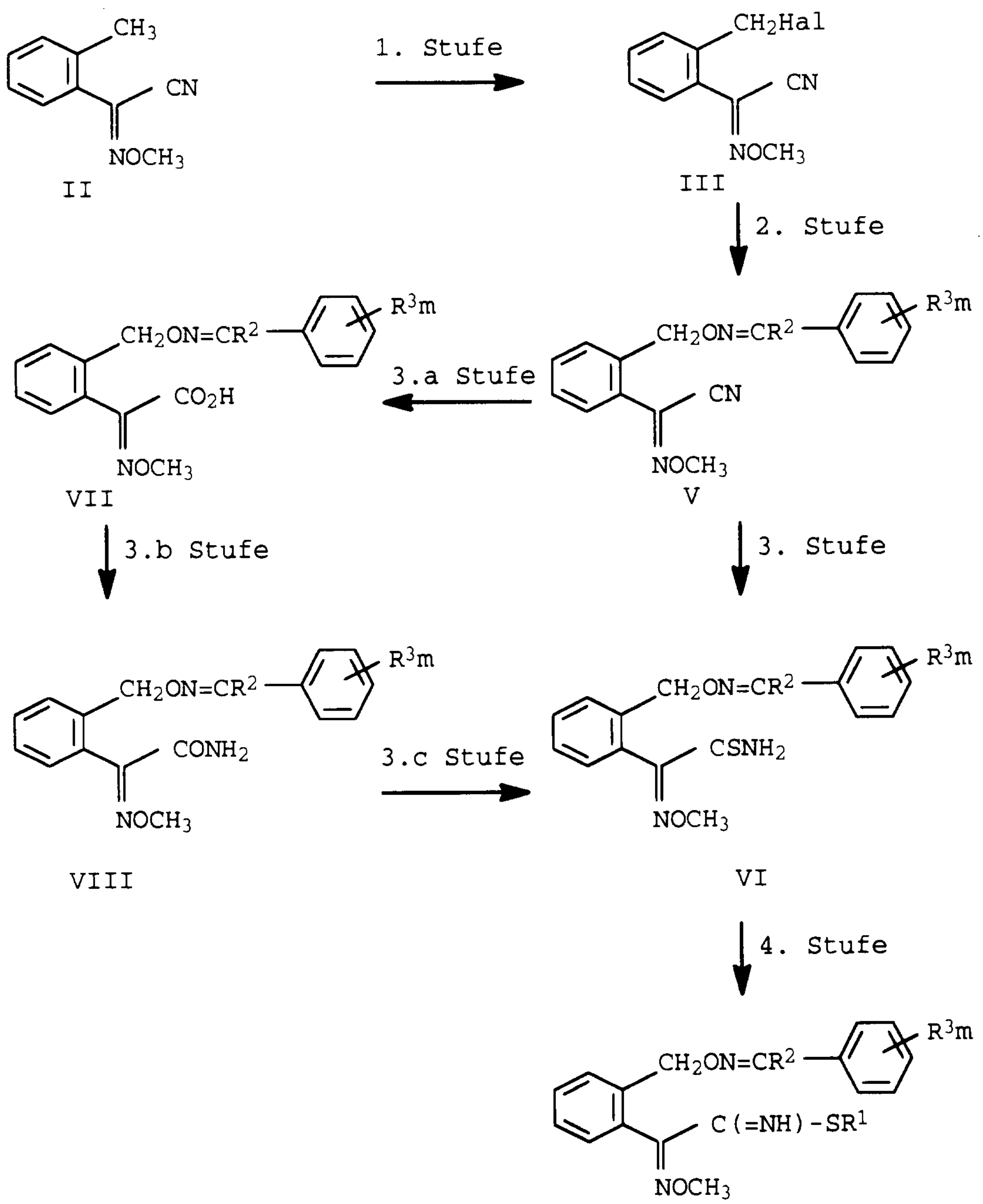


II, III, V, VII, VIII, VI, I

Die einzelnen Umsetzungen werden dabei üblicherweise wie folgt durchgeführt:

1. Herstellung des Benzylhalogenids III (EP Anm. Nr. 93 113 372.6)

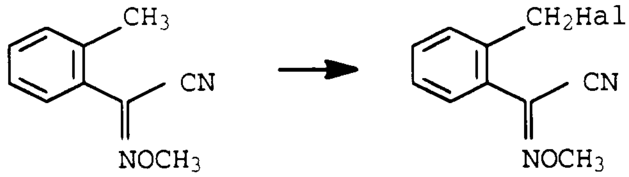

II III

Hal in der Formel III steht für ein Halogenatom, insbesondere Chlor oder Brom.

Als Halogenierungsmittel finden übliche organische oder anorganische Verbindungen, z.B. Chlor, Brom oder N-Bromsuccinimid Verwendung. Vorzugsweise verwendet man N-Bromsuccinimid.

Diese Umsetzung erfolgt bei Temperaturen von 0 ° C bis 150 ° C, vorzugsweise 40 ° C bis 100 ° C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol sowie halogenierte Kohlenwasserstoffe wie Methylenchlorid, Chloroform, Chlorbenzol und Tetrachlormethan besonders bevorzugt Tetrachlormethan.

Es können auch Gemische der genannten Lösungsmittel verwendet werden.

Die Halogenierungsmittel werden im allgemeinen äquimolar, im Überschuß oder gegebenenfalls als Lösungsmittel verwendet.

2. Herstellung des Oximethers V (vgl. EP-A 463 488, EP-A 460 575, EP-A 472 300)

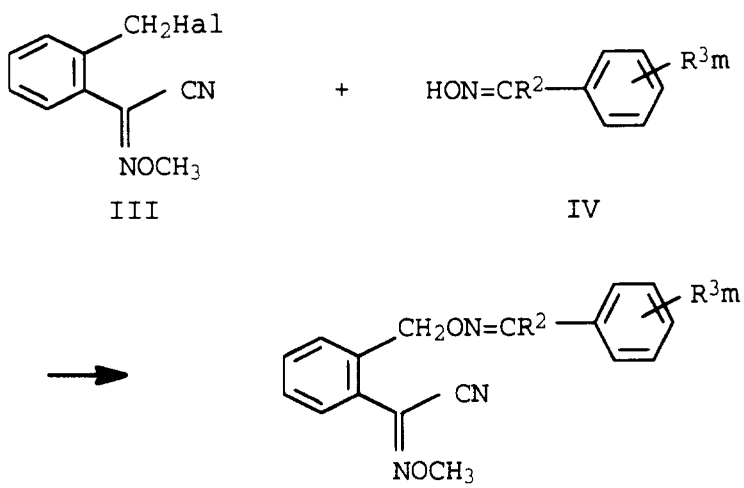

III + IV

V

Diese Umsetzung von III mit IV erfolgt üblicherweise bei Temperaturen von 0 ° C bis 100 ° C, vorzugsweise 15 ° C bis 60 ° C in einem inerten organischen Lösingsmittel in Gegenwart einer Base.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Acetonitril, Dimethylformamid und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriummethanolat, Natriumethanolat und Kaliumhydroxid.

Die Basen werden im allgemeinen äquimolar eingesetzt, sie können aber auch im Überschuß oder gegebenenfalls als Lösungsmittel verwendet werden.

Die Edukte III und IV werden im allgemeinen in äquimolaren Mengen miteinander umgesetzt. Es kann für die Ausbeute vorteilhaft sein, IV in einem Über- oder Unterschuß bezogen auf III einzusetzen.

Die für die Umsetzung benötigten Verbindungen IV sind aus der Vorliteratur bekannt (vgl. Houben-Weyl, Bd. E14b, S. 287-367) oder können analog den dort beschriebenen Verfahren hergestellt werden.

3. Herstellung des Thiocarbonsäureamids VI (vgl. Houben-Weyl, Bd. ES, S. 1253-1255)

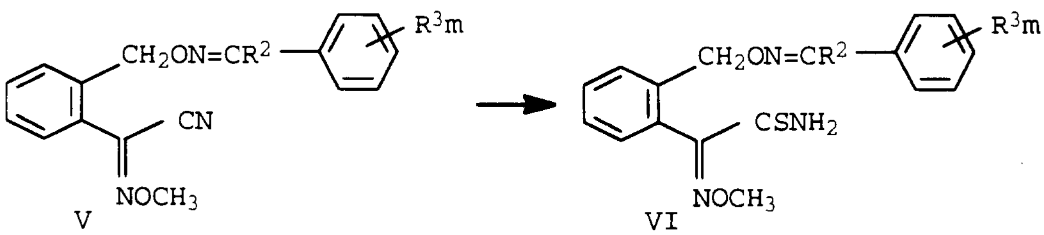

V VI

Diese Umsetzung mit Schwefelwasserstoff erfolgt üblicherweise bei Temperaturen von 10 °C bis 180 °C, vorzugsweise 20 °C bis 80 °C in einem inerten organischen Lösingsmittel in Gegenwart einer Base.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Dimethylformamid und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallcarbonate wie Lithiumcarbonat und Calziumcarbonat sowie Alkalimetallhydrogencarbonate wie Natriumhydrogencarbonat, Alkalimetall- und Erdalkallmetallalkoholate wie Natriummethanolat, Natriumethanolat, Kaliumethanolat, Kalium- tert.-Butanolat und Dimethoxymagnesium außerdem organische Basen,
z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Diethylamin, Triethylamin, Pyridin, Kaliumcarbonat, Natriumcarbonat, Kaliumhydroxid oder Natriumhydroxid.

Die Basen werden im allgemeinen in katalytischen Mengen eingesetzt, sie können aber auch äquimolar, im Überschuß oder gegebenenfalls als Lösungsmittel verwendet werden.

Neben der vorstehend beschriebenen direkten Umsetzung von V zu VI ist es auch möglich, V zunächst in die Carbonsäure VII zu überführen und VII anschließend über das Carbonsäureamid VIII in das entsprechende Thiocarbonsäureamid VI umzuwandeln.

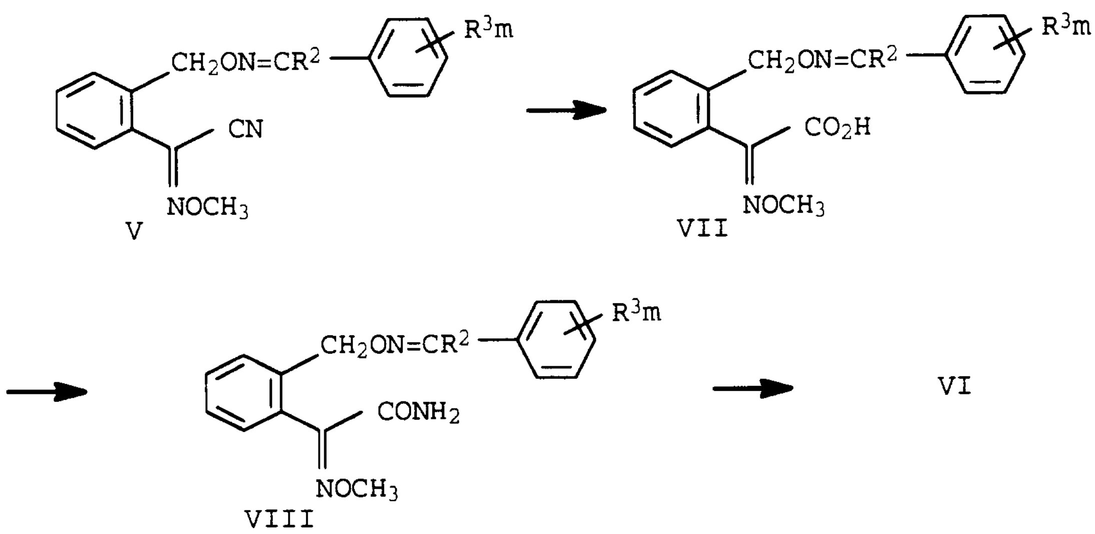

V VII VIII VI

Die Hydrolyse von V nach VII erfolgt üblicherweise durch Umsetzung mit wäßriger Säure oder Base ggf. unter Zusatz eines inerten Lösungsmittels bei 20 °C bis 100 °C (vgl. Organikum 19. Aufl., S. 441-444 (1993).

Zur Herstellung von VIII setzt man die Carbonsäure VII im allgemeinen in einem inerten Lösungsmittel bei 0 °C bis 100 °C mit Ammoniak, ggf. unter Verwendung eines Aktivierungsreagens um (vgl. Houben-Weyl, Bd. E5, S. 941-1044).

Die weitere Umsetzung von VIII zu VI erfolgt in an sich bekannter Weise mit einem Schwefelungsmittel in einem inerten Lösungsmittel bei 50 °C bis 150 °C (vgl. Houben-Weyl, Bd. E5, S. 1242-1245).

4. Herstellung des Thioimidats I

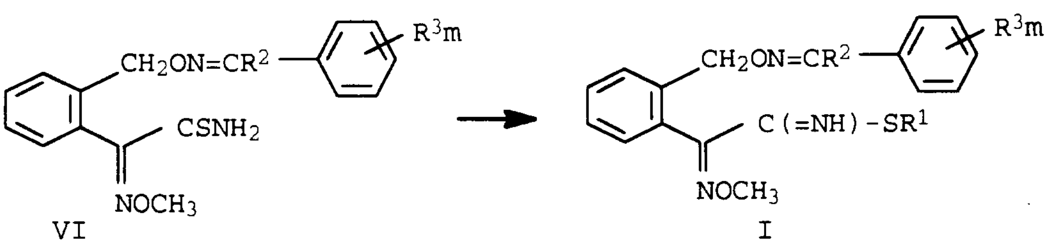

VI I

Aus den Thiocarbonsäureamiden VI erhält man die Thioimidate I üblicherweise durch Umsetzung mit einem Alkylierungsmittel in einem inerten organischen Lösungsmittel in Gegenwart einer Base. Als Alkylierungsmittel finden im allgemeinen Alkylhalogenide, insbesondere -chloride, -bromide und -iodide, oder Alkylsulfonate Verwendung.

Geeinete Lösungsmittel sind insbesondere Toluol, Dichlormethan, Dieethylether, Dioxan, Tetrahydrofuran, Dimethylformamid und Dimethylsulfoxid.

Bevorzugte Basen sind Kaliumhydroxid, Kaliumcarbonat, Natriumcarbonat, Natriumhydrid, Natriummethylat, Natrium-ethylat und Kalium-tert.-butylat.

Daneben eignen sich als Alkylierungsmittel auch Trialkyloxoniumsalze, insbesondere Trialkyloxoniumtetrafluoroborat. Geeignete Lösungsmittel sind hierbei insbesondere Toluol, Dichlormethan, Diethylether, Tetrahydrofuran und Dioxan. Eine derartige Umsetzung ist beispielsweise aus EP-A 528 681 bekannt.

Bei dem vorstehend geschilderten Verfahren zur Herstellung der Verbindungen I ist es im allgemeinen unerheblich, ob zunächst die Gruppierung -CN in die gewünschte Gruppe ($-C(=NH)-SR^1$) überführt wird oder ob zunächst die toluolische Methylgruppe umgewandelt wird. Entsprechend können die Umwandlungen in beliebiger Reihenfolge vorgenommen werden.

In den vorstehenden Umsetzungen finden insbesondere bevorzugt die eingangs definierten Verbindungen IX als Zwischenprodukte Verwendung. Die entsprechenden Verbindungen, in denen die -C=N-Doppelbindung in Bezug auf die $-OCH_3$ Gruppe und den Rest -Y "E"-konfiguriert ist, sind, z.T. aus der Literatur bekannt (EP-A 477 631, EP-A 463 488, EP Anm. Nr. 93 113 372.6) oder können nach den dort beschriebenen Verfahren erhalten werden.

Die aus den Zwischenprodukten IX erhältlichen Verbindungen I ("Z"-konfiguriert in Bezug auf die $-OCH_3$ Gruppe und den - $C(=NH)-SR^1$ Rest) stellen außerdem wertvolle Zwischenprodukte für die Herstellung der bekannten Wirkstoffe der Formel X

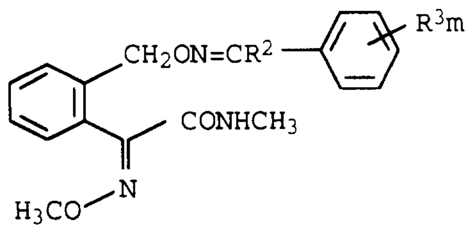

X

dar, in denen die Reste $R^2$ und $R^3$ und der Index m die vorstehende Bedeutung haben.

Man erhält diese Verbindungen X beispielsweise in der im folgenden Reaktionsschema angegebenen Weise.

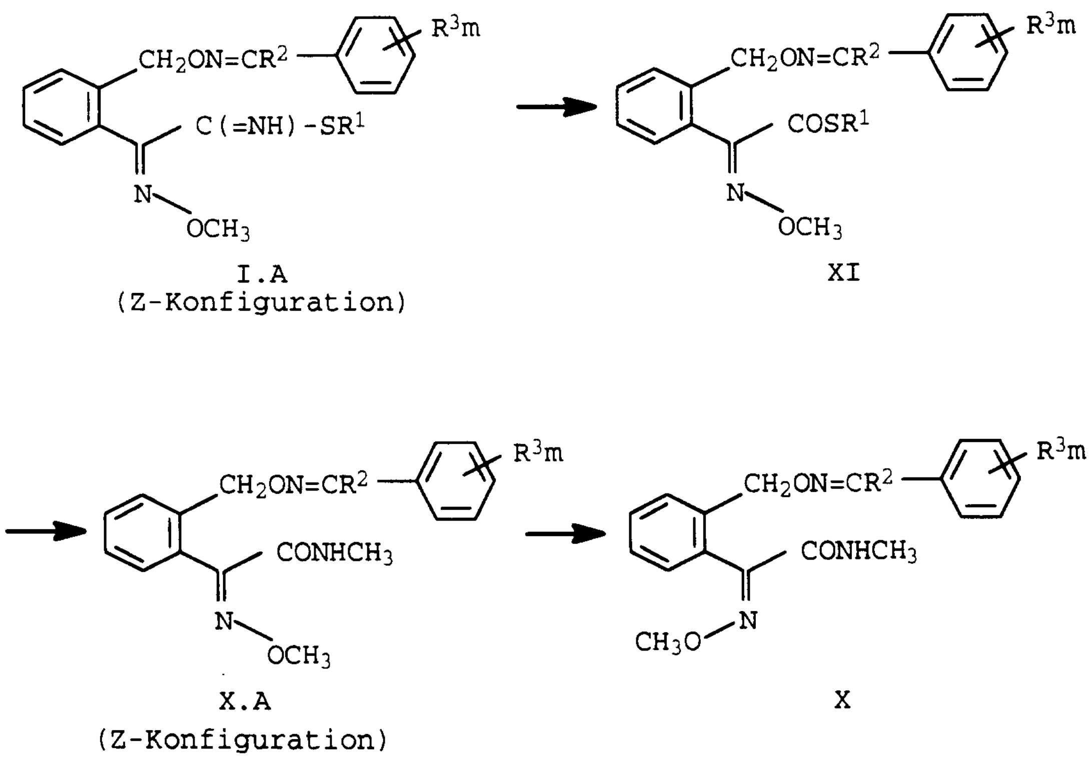

I.A
(Z-Konfiguration)

XI

X.A
(Z-Konfiguration)

X

Die Hydrolyse der Thioimidate I.A erfolgt bei 0°C bis 100°C, vorzugsweise 0°C bis 50°C, insbesondere 0°C bis 30°C in einem inerten Lösungsmittel in Gegenwart einer Säure (vgl. Liebigs. Anm. Chem. 768 (1976); Can. J. Chem. 48, 3662 (1970)).

Geeignete Lösungsmittel sind Toluol, Diethylether, Dioxan, Tetrahydrofuran, Methanol, Ethanol und Dimethylformamid.

Der so erhaltene Thioester XI wird anschließend bei 0°C bis 60°C, vorzugsweise 0°C bis 30°C, insbesondere 0°C bis 25°C in einem inerten organischen Lösungsmittel ggf. in Gegenwart von Wasser mit Methylamin umgesetzt.

Aus den so erhaltenen Z-konfigurierten Verbindungen X.A erhält man die gewünschten E-konfigurierten Wirkstoffe durch Zusatz von Säure oder photochemisch.

Die Verbindung I ist wegen des basischen Charakters der NH-Gruppierung in der Lage, mit anorganischen oder organischen Säuren oder mit Metallionen Salze oder Addukte zu bilden.

Beispiele für anorganische Säuren sind Halogenwasserstoffsäuren wie Fluorwasserstoff, Chlorwasserstoff, Bromwasserstoff und Jodwasserstoff, Schwefelsäure, Phosphorsäure und Salpetersäure.

Als organischen Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche eine oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder -diphosphonsäuren (aromatische Reste wie Phenyl und Naphthyl welche eine oder zwei Phosphorsäurereste tragen), wobei die Alkyl- bzw. Arylreste weitere Substituenten tragen können, z.B. p-Toluolsulfonsäure, Salizylsäure, p-Aminosalizylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der Nebengruppen der vierten Periode. Die Metalle können dabei in den verschiedenen ihnen zukommenden Wertigkeiten vorliegen.

Bei der eingangs angegebenen Definition der Verbindungen I und IX wurden Sammelbegriffe verwendet, die allgemein repräsentativ für die folgenden Substituenten stehen:

**Halogen:** Fluor, Chlor, Brom und Jod;

**Alkyl:** geradkettige oder verzweigte Alkylgruppen mit 1 bis 3 bzw 6 Kohlenstoffatomen, z.B. $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

**Alkylamino:** eine Aminogruppe, welche eine geradkettige oder verzweigte Alkylgruppe mit 1 bis 6 Kohlenstoffatomen wie vorstehend genannt trägt;

**Dialkylamino:** eine Aminogruppe, welche zwei voneinander unabhängige, geradkettige oder verzweigte Alkylgruppen mit jeweils 1 bis 6 Kohlenstoffatomen wie vorstehend genannt trägt;

**Halogenalkyl:** geradkettige oder verzweigte Alkylgruppen mit 1 bis 6 Kohlenstoffatomen, wobei diese in Gruppen teilweise oder vollständig die Wasserstoffatome durch Halogenatome wie vorstehend genannt ersetzt sein können, z.B. $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;

**Alkoxy:** geradkettige oder verzweigte Alkylgruppen mit 1 bis 6 Kohlenstoffatomen wie vorstehend genannt, welche über ein Sauerstoffatom (-O-) an das Gerüst gebunden sind;

**Alkoxyalkyl:** geradkettige oder verzweigte Alkylgruppen mit 1 bis 6 Kohlenstoffatomen wie vorstehend genannt, welche in einer beliebigen Position eine geradkettige oder verzweigte Alkoxygruppe mit 1 bis 6 Kohlenstoffatomen wie vorstehend genannt tragen;

**Halogenalkoxy:** geradkettige oder verzweigte Alkoxygruppen mit 1 bis 6 Kohlenstoffatomen wie vorstehend genannt, wobei in diesen Gruppen teilweise oder vollständig die Wasserstoffatome durch Halogenatome wie vorstehend genannt ersetzt sein können, z.B. $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;

**Alkylthio:** geradkettige oder verzweigte Alkylgruppen mit 1 bis 6 Kohlenstoffatomen wie vorstehend genannt, welche über ein Schwefelatom (-S-) an das Gerüst gebunden sind, z.B. $C_1$-$C_6$-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;

**Alkenyl:** geradkettige oder verzweigte Kohlenwasserstoffgruppen mit 2 bis 6 Kohlenstoffatomen und einer Doppelbindung in einer beliebigen Position, z.B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl- 1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3- butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1- butenyl, 2-Ethyl-2-butenyl, 2-Ethyl-3-butenyl, 1,1,2-Trimethyl- 2-propenyl, 1-Ethyl-1-methyl-2-propenyl, 1-Ethyl-2-methyl-1-propenyl und 1-Ethyl-2-methyl-2-propenyl;

**Alkenyloxy:** geradkettige oder verzweigte Alkenylgruppen mit 2 bis 6 Kohlenstoffatomen wie vorstehend genannt, welche über ein Sauerstoffatom (-O-) an das Gerüst gebunden sind;

**Alkinyl:** geradkettige oder verzweigte Kohlenwasserstoffgruppen mit 2 bis 6 Kohlenstoffatomen und einer Dreifachbindung in einer beliebigen Position, z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;

**Alkinyloxy:** geradkettige oder verzweigte Alkinylgruppen mit 2 bis 6 Kohlenstoffatomen wie vorstehend genannt, welche über ein Sauerstoffatom (-O-) an das Gerüst gebunden sind;

**Cycloalkyl:** monocyclische Alkylgruppen mit 3 bis 6 Kohlenstoffringgliedern, wie Cyclopropyl, Cyclobutyl, Cyclopentyl und Cyclohexyl;

**Cycloalkoxy:** monocyclische Alkylgruppen mit 3 bis 6 Kohlenstoffringgliedern wie vorstehend genannt, welche über eine Oxygruppe (-O-) an das Gerüst gebunden sind;

Besonders bevorzugt werden auch Verbindungen I, in denen $R^1$ $C_1$-$C_4$-Alkyl, insbesondere Methyl oder Ethyl bedeutet.

Daneben werden Verbindungen I bevorzugt, in denen $R^2$ für Cyclopropyl steht.

Weitere besonders bevorzugte Verbindungen I sind diejenigen, in denen $R^2$ für Methyl oder Ethyl, insbesondere Methyl, steht.

Außerdem werden Verbindungen I bevorzugt, in denen m 1, 2 oder 3 bedeutet.

Bevorzugt werden auch Verbindungen I, in denen $R^3$ für eine der folgenden Gruppen steht: $C(=O)R^a$, $C(=O)OR^a$, $OC(=O)R^a$, $C(=O)NR^aR^b$, $C(=S)NR^aR^b$, $NR^aC(=O)R^b$ und $NR^aC(=O)OR^b$, wobei $R^a$ und $R^b$ unabhängigvoneinander vorzugsweise für Wasserstoff oder Methyl steht.

Weiterhin werden Verbindungen I bevorzugt, in denen $R^3$ für eine der folgenden Gruppen steht: $CR^a=NR^b$, $C(R^a)=NOR^b$, $C(XR^a)=NOR^b$ und $N=CR^aR^b$, wobei $R^a$ und $R^b$ unabhängig voneinander vorzugsweise für Methyl oder Ethyl steht.

Daneben werden Verbindungen I bevorzugt, in denen $R^3$ für eine bis drei der folgenden Gruppen steht: Halogen, $C_1$-$C_4$-Alkyl,$C_1$-$C_4$-Halogenalkyl, $C_1$-$C_4$-Alkoxy, $C_1$-$C_4$-Halogenalkoxy, $C_1$-$C_4$-Alkylthio und/oder $C_3$-$C_6$-Cycloalkyl.

Außerdem werden Verbindungen I bevorzugt, in denen mindestens ein Rest $R^3$ für Phenyl oder Phenoxy steht.

Weitere bevorzugte Verbindungen I sind solche, in denen ein bis drei der Reste $R^3$ Halogenatome, insbesondere Chlor, bedeuten.

Bevorzugt werden auch solche Verbindungen I, in denen zwei an benachbarte C-Atome gebundene Reste $R^3$ gemeinsam für $-CH=CH-CH=CH-$ stehen.

Besonders bevorzugt werden Verbindungen I, in denen $R^1$ für Methyl oder Ethyl, $R^2$ für Methyl und $R^3$ für Halogen steht.

Im Hinblick auf ihre biologische Wirkung insbesondere bevorzugte Verbindungen I sind in den folgenden Tablellen zusammengestellt; den entsprechenden Z-konfigurierten Verbindungen kommt ebenfalls im Hinblick auf die bevorzugte Herstellung eine besondere Bedeutung zu.

Tabelle A

Verbindungen der Formel I.1, in denen die Kombination der Substituenten $R^1$ und $R^3{}_m$ für eine Verbindung jeweils einer Zeile einer Spalte der Tabelle entspricht.

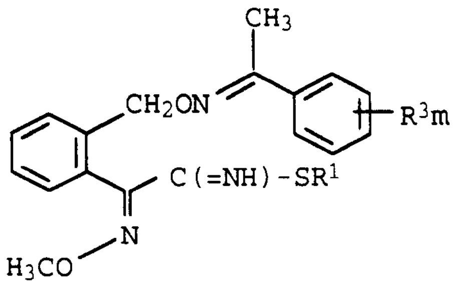

I.1

Tabelle B

Verbindungen der Formel I.2, in denen die Kombination der Substituenten $R^1$ und $R^3{}_m$ für eine Verbindung jeweils einer Zeile einer Spalte der Tabelle entspricht.

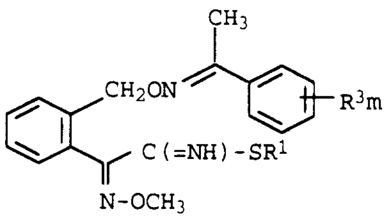

I.2

Tabelle

| Spalte 1 | | Spalte 2 | |
|---|---|---|---|
| $R^3m$ | $R^1$ | $R^3m$ | $R^1$ |
| -- (m=0) | $C_2H_5$ | 2-Cl | $C_2H_5$ |
| 3-Cl | $C_2H_5$ | 4-Cl | $C_2H_5$ |
| 3,4-$Cl_2$ | $C_2H_5$ | 3,5-$Cl_2$ | $C_2H_5$ |
| 2,3,4-$Cl_3$ | $C_2H_5$ | 2,4,5-$Cl_3$ | $C_2H_5$ |
| 2-Br | $C_2H_5$ | 3-Br | $C_2H_5$ |
| 4-Br | $C_2H_5$ | 2-$NO_2$ | $C_2H_5$ |
| 3-$NO_2$ | $C_2H_5$ | 4-$NO_2$ | $C_2H_5$ |
| 2-$CH_3$ | $C_2H_5$ | 3-$CH_3$ | $C_2H_5$ |
| 4-$CH_3$ | $C_2H_5$ | 2,4-$(CH_3)_2$ | $C_2H_5$ |
| 3,4-$(CH_3)_2$ | $C_2H_5$ | 3,5-$(CH_3)_2$ | $C_2H_5$ |
| 2,5-$(CH_3)_2$ | $C_2H_5$ | 2-$CH(CH_3)_2$ | $C_2H_5$ |
| 3-$CH(CH_3)_2$ | $C_2H_5$ | 4-$CH(CH_3)_2$ | $C_2H_5$ |
| 2-$C(CH_3)_3$ | $C_2H_5$ | 3-$C(CH_3)_3$ | $C_2H_5$ |
| 4-$C(CH_3)_3$ | $C_2H_5$ | 3-$CH_3$, 4-$C(CH_3)_3$ | $C_2H_5$ |
| 4-$CH_3$, 3-$C(CH_3)_3$ | $C_2H_5$ | 2-$OCH_3$ | $C_2H_5$ |
| 3-$OCH_3$ | $C_2H_5$ | 4-$OCH_3$ | $C_2H_5$ |
| 2-$OCH(CH_3)_2$ | $C_2H_5$ | 3-$OCH(CH_3)_2$ | $C_2H_5$ |
| 4-$OCH(CH_3)_2$ | $C_2H_5$ | 2-$OC(CH_3)_3$ | $C_2H_5$ |
| 3-$OC(CH_3)_3$ | $C_2H_5$ | 4-$OC(CH_3)_3$ | $C_2H_5$ |
| 3-$CF_3$ | $C_2H_5$ | 4-$CF_3$ | $C_2H_5$ |
| 3-$OCF_3$ | $C_2H_5$ | 4-$OCF_3$ | $C_2H_5$ |
| 3-$SC(CH_3)_3$ | $C_2H_5$ | 4-$SC(CH_3)_3$ | $C_2H_5$ |
| 3-$C(CH_3)=NOCH_3$ | $C_2H_5$ | 4-$C(CH_3)=NOCH_3$ | $C_2H_5$ |
| 2-Cl | $(CH_2)_2CH_3$ | 3-Cl | $(CH_2)_2CH_3$ |
| 4-Cl | $(CH_2)_2CH_3$ | 3,4-$Cl_2$ | $(CH_2)_2CH_3$ |

| Spalte 1 | | Spalte 2 | |
|---|---|---|---|
| $R^3m$ | $R^1$ | $R^3m$ | $R^1$ |
| 3,5-$Cl_2$ | $(CH_2)_2CH_3$ | 2,3,4-$Cl_3$ | $(CH_2)_2CH_3$ |
| 2,4,5-$Cl_3$ | $(CH_2)_2CH_3$ | 2-Br | $(CH_2)_2CH_3$ |
| 3-Br | $(CH_2)_2CH_3$ | 4-Br | $(CH_2)_2CH_3$ |
| 2-$NO_2$ | $(CH_2)_2CH_3$ | 3-$NO_2$ | $(CH_2)_2CH_3$ |
| 4-$NO_2$ | $(CH_2)_2CH_3$ | 2-$CH_3$ | $(CH_2)_2CH_3$ |
| 3-$CH_3$ | $(CH_2)_2CH_3$ | 4-$CH_3$ | $(CH_2)_2CH_3$ |
| 2,4-$(CH_3)_2$ | $(CH_2)_2CH_3$ | 3,4-$(CH_3)_2$ | $(CH_2)_2CH_3$ |
| 3,5-$(CH_3)_2$ | $(CH_2)_2CH_3$ | 2,5-$(CH_3)_2$ | $(CH_2)_2CH_3$ |
| 2-$CH(CH_3)_2$ | $(CH_2)_2CH_3$ | 3-$CH(CH_3)_2$ | $(CH_2)_2CH_3$ |
| 4-$CH(CH_3)_2$ | $(CH_2)_2CH_3$ | 2-$C(CH_3)_3$ | $(CH_2)_2CH_3$ |
| 3-$C(CH_3)_3$ | $(CH_2)_2CH_3$ | 4-$C(CH_3)_3$ | $(CH_2)_2CH_3$ |
| 3-$CH_3$, 4-$C(CH_3)_3$ | $(CH_2)_2CH_3$ | 4-$CH_3$, 3-$C(CH_3)_3$ | $(CH_2)_2CH_3$ |
| 2-$OCH_3$ | $(CH_2)_2CH_3$ | 3-$OCH_3$ | $(CH_2)_2CH_3$ |
| 4-$OCH_3$ | $(CH_2)_2CH_3$ | 2-$OCH(CH_3)_2$ | $(CH_2)_2CH_3$ |
| 3-$OCH(CH_3)_2$ | $(CH_2)_2CH_3$ | 4-$OCH(CH_3)_2$ | $(CH_2)_2CH_3$ |
| 2-$OC(CH_3)_3$ | $(CH_2)_2CH_3$ | 3-$OC(CH_3)_3$ | $(CH_2)_2CH_3$ |
| 4-$OC(CH_3)_3$ | $(CH_2)_2CH_3$ | 3-$CF_3$ | $(CH_2)_2CH_3$ |
| 4-$CF_3$ | $(CH_2)_2CH_3$ | 3-$OCF_3$ | $(CH_2)_2CH_3$ |
| 4-$OCF_3$ | $(CH_2)_2CH_3$ | 3-$SC(CH_3)_3$ | $(CH_2)_2CH_3$ |
| 4-$SC(CH_3)_3$ | $(CH_2)_2CH_3$ | 3-$C(CH_3)=NOCH_3$ | $(CH_2)_2CH_3$ |
| 4-$C(CH_3)=NOCH_3$ | $(CH_2)_2CH_3$ | 2-Cl | $CH_3$ |
| 3-Cl | $CH_3$ | 4-Cl | $CH_3$ |
| 3,4-$Cl_2$ | $CH_3$ | 3,5-$Cl_2$ | $CH_3$ |
| 2,3,4-$Cl_3$ | $CH_3$ | 2,4,5-$Cl_3$ | $CH_3$ |
| 2-Br | $CH_3$ | 3-Br | $CH_3$ |
| 4-Br | $CH_3$ | 2-$NO_2$ | $CH_3$ |
| 3-$NO_2$ | $CH_3$ | 4-$NO_2$ | $CH_3$ |
| 2-$CH_3$ | $CH_3$ | 3-$CH_3$ | $CH_3$ |
| 4-$CH_3$ | $CH_3$ | 2,4-$(CH_3)_2$ | $CH_3$ |
| 3,4-$(CH_3)_2$ | $CH_3$ | 3,5-$(CH_3)_2$ | $CH_3$ |
| 2,5-$(CH_3)_2$ | $CH_3$ | 2-$CH(CH_3)_2$ | $CH_3$ |
| 3-$CH(CH_3)_2$ | $CH_3$ | 4-$CH(CH_3)_2$ | $CH_3$ |
| 2-$C(CH_3)_3$ | $CH_3$ | 3-$C(CH_3)_3$ | $CH_3$ |
| 4-$C(CH_3)_3$ | $CH_3$ | 3-$CH_3$, 4-$C(CH_3)_3$ | $CH_3$ |
| 4-$CH_3$, 3-$C(CH_3)_3$ | $CH_3$ | 2-$OCH_3$ | $CH_3$ |
| 3-$OCH_3$ | $CH_3$ | 4-$OCH_3$ | $CH_3$ |
| 2-$OCH(CH_3)_2$ | $CH_3$ | 3-$OCH(CH_3)_2$ | $CH_3$ |
| 4-$OCH(CH_3)_2$ | $CH_3$ | 2-$OC(CH_3)_3$ | $CH_3$ |

| Spalte 1 | | | Spalte 2 | |
|---|---|---|---|---|
| $R^3m$ | $R^1$ | | $R^3m$ | $R^1$ |
| 3-OC(CH$_3$)$_3$ | CH$_3$ | | 4-OC(CH$_3$)$_3$ | CH$_3$ |
| 3-CF$_3$ | CH$_3$ | | 4-CF$_3$ | CH$_3$ |
| 3-OCF$_3$ | CH$_3$ | | 4-OCF$_3$ | CH$_3$ |
| 3-SC(CH$_3$)$_3$ | CH$_3$ | | 4-SC(CH$_3$)$_3$ | CH$_3$ |
| 3-C(CH$_3$)=NOCH$_3$ | CH$_3$ | | 4-C(CH$_3$)=NOCH$_3$ | CH$_3$ |
| 2-Cl | (CH$_2$)$_3$CH$_3$ | | 3-Cl | (CH$_2$)$_3$CH$_3$ |
| 4-Cl | (CH$_2$)$_3$CH$_3$ | | 3,4-Cl$_2$ | (CH$_2$)$_3$CH$_3$ |
| 3,5-Cl$_2$ | (CH$_2$)$_3$CH$_3$ | | 2,3,4-Cl$_3$ | (CH$_2$)$_3$CH$_3$ |
| 2,4,5-Cl$_3$ | (CH$_2$)$_3$CH$_3$ | | 2-Br | (CH$_2$)$_3$CH$_3$ |
| 3-Br | (CH$_2$)$_3$CH$_3$ | | 4-Br | (CH$_2$)$_3$CH$_3$ |
| 2-NO$_2$ | (CH$_2$)$_3$CH$_3$ | | 3-NO$_2$ | (CH$_2$)$_3$CH$_3$ |
| 4-NO$_2$ | (CH$_2$)$_3$CH$_3$ | | 2-CH$_3$ | (CH$_2$)$_3$CH$_3$ |
| 3-CH$_3$ | (CH$_2$)$_3$CH$_3$ | | 4-CH$_3$ | (CH$_2$)$_3$CH$_3$ |
| 2,4-(CH$_3$)$_2$ | (CH$_2$)$_3$CH$_3$ | | 3,4-(CH$_3$)$_2$ | (CH$_2$)$_3$CH$_3$ |
| 3,5-(CH$_3$)$_2$ | (CH$_2$)$_3$CH$_3$ | | 2,5-(CH$_3$)$_2$ | (CH$_2$)$_3$CH$_3$ |
| 2-CH(CH$_3$)$_2$ | (CH$_2$)$_3$CH$_3$ | | 3-CH(CH$_3$)$_2$ | (CH$_2$)$_3$CH$_3$ |
| 4-CH(CH$_3$)$_2$ | (CH$_2$)$_3$CH$_3$ | | 2-C(CH$_3$)$_3$ | (CH$_2$)$_3$CH$_3$ |
| 3-C(CH$_3$)$_3$ | (CH$_2$)$_3$CH$_3$ | | 4-C(CH$_3$)$_3$ | (CH$_2$)$_3$CH$_3$ |
| 3-CH$_3$, 4-C(CH$_3$)$_3$ | (CH$_2$)$_3$CH$_3$ | | 4-CH$_3$, 3-C(CH$_3$)$_3$ | (CH$_2$)$_3$CH$_3$ |
| 2-OCH$_3$ | (CH$_2$)$_3$CH$_3$ | | 3-OCH$_3$ | (CH$_2$)$_3$CH$_3$ |
| 4-OCH$_3$ | (CH$_2$)$_3$CH$_3$ | | 2-OCH(CH$_3$)$_2$ | (CH$_2$)$_3$CH$_3$ |
| 3-OCH(CH$_3$)$_2$ | (CH$_2$)$_3$CH$_3$ | | 4-OCH(CH$_3$)$_2$ | (CH$_2$)$_3$CH$_3$ |
| 2-OC(CH$_3$)$_3$ | (CH$_2$)$_3$CH$_3$ | | 3-OC(CH$_3$)$_3$ | (CH$_2$)$_3$CH$_3$ |
| 4-OC(CH$_3$)$_3$ | (CH$_2$)$_3$CH$_3$ | | 3-CF$_3$ | (CH$_2$)$_3$CH$_3$ |
| 4-CF$_3$ | (CH$_2$)$_3$CH$_3$ | | 3-OCF$_3$ | (CH$_2$)$_3$CH$_3$ |
| 4-OCF$_3$ | (CH$_2$)$_3$CH$_3$ | | 3-SC(CH$_3$)$_3$ | (CH$_2$)$_3$CH$_3$ |
| 4-SC(CH$_3$)$_3$ | (CH$_2$)$_3$CH$_3$ | | 3-C(CH$_3$)=NOCH$_3$ | (CH$_2$)$_3$CH$_3$ |
| 4-C(CH$_3$)=NOCH$_3$ | (CH$_2$)$_3$CH$_3$ | | 2-Cl | CH(CH$_3$)$_2$ |
| 3-Cl | CH(CH$_3$)$_2$ | | 4-Cl | CH(CH$_3$)$_2$ |
| 3,4-Cl$_2$ | CH(CH$_3$)$_2$ | | 3,5-Cl$_2$ | CH(CH$_3$)$_2$ |
| 2,3,4-Cl$_3$ | CH(CH$_3$)$_2$ | | 2,4,5-Cl$_3$ | CH(CH$_3$)$_2$ |
| 2-Br | CH(CH$_3$)$_2$ | | 3-Br | CH(CH$_3$)$_2$ |
| 4-Br | CH(CH$_3$)$_2$ | | 2-NO$_2$ | CH(CH$_3$)$_2$ |
| 3-NO$_2$ | CH(CH$_3$)$_2$ | | 4-NO$_2$ | CH(CH$_3$)$_2$ |
| 2-CH$_3$ | CH(CH$_3$)$_2$ | | 3-CH$_3$ | CH(CH$_3$)$_2$ |
| 4-CH$_3$ | CH(CH$_3$)$_2$ | | 2,4-(CH$_3$)$_2$ | CH(CH$_3$)$_2$ |
| 3,4-(CH$_3$)$_2$ | CH(CH$_3$)$_2$ | | 3,5-(CH$_3$)$_2$ | CH(CH$_3$)$_2$ |
| 2,5-(CH$_3$)$_2$ | CH(CH$_3$)$_2$ | | 2-CH(CH$_3$)$_2$ | CH(CH$_3$)$_2$ |

| Spalte 1 | | | Spalte 2 | |
|---|---|---|---|---|
| $R^3m$ | $R^1$ | | $R^3m$ | $R^1$ |
| 3-$CH(CH_3)_2$ | $CH(CH_3)_2$ | | 4-$CH(CH_3)_2$ | $CH(CH_3)_2$ |
| 2-$C(CH_3)_3$ | $CH(CH_3)_2$ | | 3-$C(CH_3)_3$ | $CH(CH_3)_2$ |
| 4-$C(CH_3)_3$ | $CH(CH_3)_2$ | | 3-$CH_3$, 4-$C(CH_3)_3$ | $CH(CH_3)_2$ |
| 4-$CH_3$, 3-$C(CH_3)_3$ | $CH(CH_3)_2$ | | 2-$OCH_3$ | $CH(CH_3)_2$ |
| 3-$OCH_3$ | $CH(CH_3)_2$ | | 4-$OCH_3$ | $CH(CH_3)_2$ |
| 2-$OCH(CH_3)_2$ | $CH(CH_3)_2$ | | 3-$OCH(CH_3)_2$ | $CH(CH_3)_2$ |
| 4-$OCH(CH_3)_2$ | $CH(CH_3)_2$ | | 2-$OC(CH_3)_3$ | $CH(CH_3)_2$ |
| 3-$OC(CH_3)_3$ | $CH(CH_3)_2$ | | 4-$OC(CH_3)_3$ | $CH(CH_3)_2$ |
| 3-$CF_3$ | $CH(CH_3)_2$ | | 4-$CF_3$ | $CH(CH_3)_2$ |
| 3-$OCF_3$ | $CH(CH_3)_2$ | | 4-$OCF_3$ | $CH(CH_3)_2$ |
| 3-$SC(CH_3)_3$ | $CH(CH_3)_2$ | | 4-$SC(CH_3)_3$ | $CH(CH_3)_2$ |
| 3-$C(CH_3)$=$NOCH_3$ | $CH(CH_3)_2$ | | 4-$C(CH_3)$=$NOCH_3$ | $CH(CH_3)_2$ |
| 3,4-CH=CH-CH=CH- | $CH_3$ | | 3,4-CH=CH-CH=CH- | $C_2H_5$ |

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusa7rium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und

Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:

Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat,Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;

Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;

heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazolcarbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,

N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäureanilid, N-Formyl-N-morpholin-2,2,2-trichlorethylacetal, Piperazin-1,4-diyl-bis-(1-(2,2,2-trichlor-ethyl)-formamid, 1-(3,4-Dichloranilino)-1-formylamino-2,2,2-trichlorethan , 2,6-Dimethyl-N-tridecyl-morpholin bzw. dessen Salze, 2,6-Dimethyl-N-cyclododecylmorpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H- 1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl- harnstoff, 1-(4-Chlorphenoxy)-3,3-dimethyl-1-(1H-1,2,4-triazol-1-yl)-2- butanon, 1-(4-Chlorphenoxy)-3,3-dimethyl-1-(1H-1,2,4-triazol-1-yl)-2- butanol, α-(2-Chlorphenyl)-α-(4-chlorphenyl)-5-pyrimidin-methanol, 5-Butyl-2-dimethylamino-4-hydroxy-6-methylpyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,

sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia sübterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis ecuestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus

maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. 5 Gew.-Teile der Verbindung Nr. 7 werden mit 95 Gew.-Teilen feinteiligem Kaolin innig vermischt. Man erhält auf diese Weise ein Stäubemittel, das 5 Gew.% des Wirkstoffs enthält.

II. 30 Gew.-Teile der Verbindung Nr. 11 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).

III. 10 Gew.-Teile der Verbindung Nr. 5 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).

IV. 20 Gew.-Teile der Verbindung Nr. 10 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).

V. 80 Gew.-Teile der Verbindung Nr. 5 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).

VI. Man vermischt 90 Gew.-Teile der Verbindung Nr. 7 mit 10 Gew.-Teilen N-Methyl-α-pyrrolidon und erhält eine Lösung, die zur Anwendung in Form kleinster Tropfen geeignet ist (Wirkstoffgehalt 90 Gew.%).

VII. 20 Gew.-Teile der Verbindung Nr. 11 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

VIII. 20 Gew.-Teile des Wirkstoffs Nr. 10 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Synthesebeispiele

Die in den nachstehenden Synthesebeispielen wiedergegebenen Vorschriften können unter entsprechender Abwandlung der Ausgangsverbindungen zur Gewinnung weiterer Verbindungen I benutzt werden.

Beispiel 1

Herstellung von (Z)-2-Methoxyimino-2-(2'-methylphenyl)acetonitril

188 g (1,68 mol) trockenes Kalium-tert.-butylat wurden in 2 l trockenem Toluol vorgelegt und zügig mit 200 g (1,53 mol) ortho-Methylbenzylcyanidund 173 g (1,68 mol) tert.-Butylnitrit in 200 ml Toluol versetzt. Das Gemisch erwärmte sich auf ca. 70 °C, wurde 2 h nachgerührt und mit 1 l Methyl-tert.-butylether versetzt. Das gelbe Kaliumsalz wurde abgesaugt, mit Methyl-tert.-butylether nachgewaschen und bei 50 °C im Vakuum getrocknet.

290 g (1,46 mol) dieses Salzes wurden in 2,5 l trockenem Aceton mit 20 g (0,15 mol) Kaliumcarbonat vorgelegt. Während man 234 g (1,65 mol) Jodmethan zutropfte, stieg die Temperatur auf ca. 35 °C. Man ließ über Nacht nachrühren und verteilte dann zwischen Wasser und Methyl-tert.-butylether. Die organische Phase wurde mit Wasser gewaschen, über $Na_2SO_4$ getrocknet, eingeengt und im Vakuum destilliert (Kp. = 95 - 100 °C / 0,3 - 0,1 mm). Man erhielt 229 g (78 %) der Titelverbindung als schwerbewegliche Flüssigkeit.

$^1$H-NMR ($CDCl_3$): δ = 2,53 (s, 3H); 4,22 (s, 3H); 7,2 - 7,4 (m, 3H); 7,54 (dd, 1H) ppm.

Beispiel 2

Herstellung von (Z)-2-Methoxyimino-2-(2'-brommethylphenyl)acetonitril

48 g ( 0,28 mol) (Z)-2-Methoxyimino-2-(2'-methylphenyl)acetonitril und 54 g (0,30 mol) N-Bromsuccinimid wurden in 250 ml Tetrachlorkohlenstoff vorgelegt. Es wurde 40 min mit einer Hg-Dampflampe (300 W) von außen bestrahlt. Das Succinimid wurde abgesaugt und die Lösung im Vakuum eingedamft. Es verblieben 62 g (88 %) der Titelverbindung (Reinheit laut $^1$H-NMR-Spektrum: ca. 80 %ig).

$^1$H-NMR ($CDCl_3$): δ = 4,27 (s, 3H); 4,80 (s, 2H); 7,4 - 7,5 (m, 3H); 7,69 (dd, 1H) ppm.

Beispiel 3

Herstellung von (Z)-2-Methoxyimino-2-[2'-(E)-(1''-(3''',5'''-dichlorphenyl)-1''-methyl)iminooxymethyl]-phenylacetonitril

6 g (0,25 mol) Natriumhydrid wurden in 800 ml Acetonitril unter Schutzgas vorgelegt und bei Raumtemperatur portionsweise 47 g (0,23 mol) 3,5-Dichloracetophenonoxim zugegeben. Es wurde 1 Stunde auf 70 °C erwärmt. Nach Abkühlen auf 50 °C wurden 74 g (0,23 mol) (Z)-2-Methoxyimino-2-(2'-brommethylphenyl)acetonitril in 200 ml Acetonitril zugetropft. Anschließend ließ man 8 Stunden nachrühren. Man goß auf kalte 2M Salzsäure und extrahierte mit Methyl-tert.-Butylether. Die vereinigten organischen Phasen wurden mit Wasser gewaschen, über $Na_2SO_4$ getrocknet und eingeengt. Nach säulenchromatographischer Reinigung an Kieselgel (Methyl-tert.-Butylether/n-Hexan) und Verreiben mit Diisopropylether erhielt man 55 g (64 % Ausbeute) der Titelverbindung als hellgelbe Kristalle vom Schmelzpunkt 73 bis 75 °C.

$^1$H-NMR ($CDCl_3$): δ = 2,26 (s, 3H); 4,22 (s, 3H); 5,49 (s, 2H); 7,32 - 7,69 (m, 7H) ppm.

Beispiel 4

Herstellung von (Z)-2-Methoxyimino-2-[2'-(E)-(1''-(3''',5'''-dichlorphenyl)-1''-methyl)iminooxymethyl]-phenylessigsäurethioamid

Zu einer Lösung von 20 g (0,053 mol) (Z)-2-Methoxyimino-2-[2'-(E)-(1''-(3''',5'''-dichlorphenyl)-1''-methyl)iminooxymethyl]phenylacetonitril und 2,0 g (0,027 mol) Diethylamin in 200 ml Dimethylformamid wurde bei 50 °C bis zur Sättigung Schwefelwasserstoff eingegast. Es wurden 30 Minuten nachgerührt. Anschließend goß man auf Wasser und extrahierte mit Methyl-tert.-Butylether. Die vereinigten organischen Phasen wurden mit Wasser gewaschen, über $Na_2SO_4$ getrocknet und eingeengt. Nach Verreiben mit Methyl-tert.-Butylether/n-Hexan erhielt man 21,7 g (100 % Ausbeute) der Titelverbindung als hellgelbe Kristalle vom Schmelzpunkt 108 bis 110 °C.

$^1$H-NMR ($CDCl_3$): δ = 2,21 (s, 3H); 4,05 (s, 3H); 5,41 (s, 2H); 7,33 - 7,58 (m, 7H); 7,83 (brs, 1H) ppm.

Beispiel 5

Herstellung von S-Methyl-(Z)-2-Methoxyimino-2-[2'-(E)-(1''-(3''',5'''-dichlorphenyl)-1''-methyl)-iminooxymethyl]phenylthioacetimidat

Zu einer Lösung von 8,2 g (0,020 mol) Z-2-Methoxyimino-2-[2'-(E)-(1''-(3''',5'''-dichlorphenyl)-1''-methyl)iminooxymethyl]phenylessigsäurethioamid in 60 ml Methylenchlorid gab man 3,0 g (0,020 mol) Trimethyloxoniumtetrafluoroborat und ließ die Mischung 1 Stunde bei Raumtemperatur rühren. Es wurde mit Wasser verdünnt und mit Methylenchlorid extrahiert. Nach Trocknen ($Na_2SO_4$) und Einrotieren der organischen Phase sowie anschließendem Verreiben mit Methyl-tert.-Butylether/n-Hexan erhielt man 6,3 g (74 % Ausbeute) der Titelverbindung als hellgelbe Kristalle vom Schmelzpunkt 81 bis 83 °C.

$^1$H-NMR ($CDCl_3$): δ = 2,25 (s, 3H); 2,42 (s, 3H); 4,04 (s, 3H); 5,46 (s, 2H); 7,32 - 7,51 (m, 7H) ppm.

Beispiel 6

Herstellung von (E,E)-2-Methoxyimino-2-[2'-(1''-(3''',5'''-dichlorphenyl)-1''-methyl)iminooxymethyl]-phenylessigsäurethioamid

Zu einer Lösung von 8,2 g (0,020 mol) (Z)-2-Methoxyimino-2-[2'-(E)-(1''-(3''',5'''-dichlorphenyl)-1''-methyl)iminooxymethyl]phenylessigsäurethioamid in 100 ml Toluol gab man 5 ml einer gesättigten etherischen Chlorwasserstofflösung und ließ bei Raumtemperatur 16 Stunden stehen. Nach Zusatz von Methyl-tert.-Butylether wurde mit gesättigter $NaHCO_3$-Lösung und anschließend mit Wasser neutralgewaschen, die organische Phase abgetrennt, über $Na_2SO_4$ getrocknet und eingeengt. Man erhielt 5,4 g (66 % Ausbeute) der Titelverbindung als hellgelbe Kristalle vom Schmelzpunkt 138 bis 140 °C.
$^1$H-NMR ($CDCl_3$): $\delta$ = 2,18 (s, 3H); 3,99 (s, 3H); 5,10 (s, 2H); 7,12 - 7,51 (m, 7H); 8,13 (br, 1H) ppm.

Beispiel 7

Herstellung von S-Methyl-(E,E)-2-Methoxyimino-2-[2'-(1''-(3''',5'''-dichlorphenyl)-1''-methyl)iminooxymethyl]-phenylthioacetimidat

Zu einer Lösung von 4,5 g (1 mmol) (E,E)-2-Methoxyimino-2-[2'-(1''-(3''',5'''-dichlorphenyl)-1''-methyl)-iminooxymethyl]phenylessigsäurethioamid in 40 ml Methylenchlorid gab man 1,6 g (11 mmol) Trimethyloxoniumtetrafluoroborat und ließ die Mischung 1 Stunde bei Raumtemperatur rühren. Es wurde mit Wasser verdünnt und mit Methylenchlorid extrahiert. Nach Trocknen ($Na_2SO_2$) und Einrotieren der organischen Phase erhielt man 4,4 g (94 % Ausbeute) der Titelverbindung als hellgelbes Öl.
$^1$H-NMR ($CDCl_3$): $\delta$ = 2,14 (s, 3H); 2,37 (s, 3H); 3,99 (s, 3H); 5,07 (s, 2H); 7,10 - 7,54 (m, 7H) ppm.

Beispiel 8

Herstellung von (Z)-2-Methoxyimino-2-[2'-(E)-(1''-(4'''-chlorphenyl)-1''-methyl)iminooxymethyl]-phenylacetonitril

5,6 g (0,23 mol) Natriumhydrid wurden in 1000 ml Acetonitril unter Schutzgas vorgelegt und bei Raumtemperatur portionsweise 36 g (0,21 mol) 4-Chloracetophenonoxim zugegeben. Es wurde 1 Stunde auf 70 °C erwärmt. Nach Abkühlen auf 50 °C wurden 67 g (0,21 mol) (Z)-2-Methoxyimino-2-(2'-brommethylphenyl)acetonitril in 200 ml Acetonitril zugetropft. Anschließend ließ man 8 Stunden nachrühren. Man goß auf kalte 2M Salzsäure und extrahierte mit Methyl-tert.-Butylether. Die vereinigten organischen Phasen wurden mit Wasser gewaschen, über $Na_2SO_4$ getrocknet und eingeengt. Nach säulenchromatographischer Reinigung an Kieselgel (Methyl-tert.-Butylether/n-Hexan) und Verreiben mit Diisopropylether erhielt man 57 g (79 % Ausbeute) der Titelverbindung als hellgelbes Pulver vom Schmelzpunkt 54 bis 56 °C.
$^1$H-NMR ($CDCl_3$): $\delta$ = 2,28 (s, 3H); 4,20 (s, 3H); 5,47 (s, 2H); 7,30 - 7,69 (m, 8H) ppm.

Beispiel 9

Herstellung von (Z)-2-Methoxyimino-2-[2'-(E)-(1''-(4'''-chlorphenyl)-1''-methyl)iminooxymethyl]-phenylessigsäurethioamid

Zu einer Lösung von 57 g (0,167 mol) (Z)-2-Methoxyimino-2-[2'-(E)-(1''-(4'''-chlorphenyl)-1''-methyl)-iminooxymethyl]phenylacetonitril und 5,8 g (0,079 mol) Diethylamin in 500 ml Dimethylformamid wurde bei 50 °C bis zur Sättigung Schwefelwasserstoff eingegast. Es wurden 30 Minuten nachgerührt. Anschließend goß man auf Wasser und extrahierte mit Methylenchlorid. Die vereinigten organischen Phasen wurden mit Wasser gewaschen, über $Na_2SO_4$ getrocknet und eingeengt. Nach Verreiben mit Methyl-tert.-Butylether/n-Hexan erhielt man 60 g (96 % Ausbeute) der Titelverbindung als hellgelbes Pulver vom Schmelzpunkt 65 bis 67 °C.
$^1$H-NMR ($CDCl_3$) : $\delta$ = 2,23 (s, 3H); 4,03 (s, 3H); 5,40 (s, 2H); 7,29 - 7,64 (m, 8H); 7,81 (brs, 1H) ppm.

Beispiel 10

Herstellung von S-Methyl-(Z)-2-Methoxyimino-2-[2'-(E)-(1''-(4'''-chlorphenyl)-1''-methyl)iminooxymethyl]-phenylthioacetimidat

Zu einer Lösung von 10 g (0,027 mol) Z-2-Methoxyimino-2-[2'-(E)-(1''-(4'''-chlorphenyl)-1''-methyl)-iminooxymethyl]phenylessigsäurethioamid in 50 ml Methylenchlorid gab man 3,9 g (0,027 mol) Trimethyloxoniumtetrafluoroborat und ließ die Mischung 1 Stunde bei Raumtemperatur rühren. Es wurde mit Wasser verdünnt und mit Methyl-tert.-Butylether extrahiert. Nach Trocknen ($Na_2SO_4$) und Einrotieren der organischen Phase sowie anschließendem Verreiben mit n-Hexan erhielt man 7,6 g (73 % Ausbeute) der Titelverbindung als hellgelbe Kristalle vom Schmelzpunkt 56 bis 58 °C.

$^1$H-NMR ($CDCl_3$): δ = 2,27 (s, 3H); 2,42 (s, 3H); 4,04 (s, 3H); 5,42 (s, 2H); 7,30 - 7,59 (m, 8H); 9,58 (sbr, 1H) ppm.

Beispiel 11

Herstellung von (Z)-2-Methoxyimino-2-[2'-(E)-(1''-(4'''-chlorphenyl)-1''-methyl)iminooxymethyl]-phenylthioessigsäure-S-methylester

Zu einer Lösung von 5,0 g (0,013 mol) S-Methyl-(Z)-2-Methoxyimino-2-[2'-(E)-(1''-(4'''-chlorphenyl)-1''-methyl)iminooxymethyl]phenylthioacetimidat in 200 ml Tetrahydrofuran gab man 25 ml 10 %ige Salzsäure und ließ bei Raumtemperatur stehen. Es wurde mit Wasser verdünnt und mit Methyl-tert.-Butylether extrahiert. Die vereinigten organischen Phasen wurden mit gesättigter $NaHCO_3$-Lösung und anschließend mit Wasser neutralgewaschen, die organische Phase abgetrennt, über $Na_2SO_4$ getrocknet und eingeengt. Nach Filtration über Kieselgel (Methyl-tert.-Butylether/n-Hexan) erhielt man 4,0 g (80 % Ausbeute) der Titelverbindung als gelbes Öl.

$^1$H-NMR ($CDCl_3$): δ = 2,28 (s, 3H); 2,43 (s, 3H); 4,03 (s, 3H); 5,45 (s, 2H); 7,30 - 7,61 (m, 8H) ppm.

Beispiel 12

Herstellung von (Z)-2-Methoxyimino-2-[2'-(E)-(1''-(4'''-chlorphenyl)-1''-methyl)iminooxymethyl]-phenylessigsäuremonomethylamid

2,0 g (5,1 mmol) (Z)-2-Methoxyimino-2-[2-(E)-(1''-(4'''-chlorphenyl)-1''-methyl)iminooxymethyl]-phenylthioessigsäure-S-methylester wurden in 200 ml Tetrahydrofuran gelöst, mit 50 ml 40 %iger wäßriger Monomethylaminlösung versetzt und 2 Stunden bei Raumtemperatur gerührt. Anschließend wurde eingeengt, der Rückstand in Methyl-tert.-butylether aufgenommen, die organische Phase mit Wasser extrahiert, über Natriumsulfat getrocknet und erneut eingeengt. Man erhielt 1,8 g (94 % Ausbeute) der Titelverbindung als beigefarbenes Pulver vom Schmelzpunkt 111 bis 113 °C.

$^1$H-NMR ($CDCl_3$): δ = 2,23 (s, 3H); 2,80 (d, 3H); 4,04 (s, 3H); 5,39 (s, 2H); 6,69 (s, br, 1H); 7,30 - 7,55 (m, 8H) ppm.

Beispiel 13

Herstellung von (E,E)-2-Methoxyimino-2-[2'-(1''-(4'''-chlorphenyl)-1''-methyl)iminooxymethyl]-phenylessigsäuremonomethylamid

Zu einer Lösung von 8,4 g (0,022 mol) (Z)-2-Methoxyimino-2-[2'-(E)-(1''-(4'''-chlorphenyl)-1''-methyl)-iminooxymethyl]phenylessigsäuremonomethylamidin 300 ml Toluol gab man 50 ml einer gesättigten etherischen Chlorwasserstofflösung und ließ bei Raumtemperatur 4 Stunden stehen. Nach Zusatz von Methyl-tert.-Butylether wurde mit gesättigter $NaHCO_3$-Lösung und anschließend mit Wasser neutralgewaschen, die organische Phase abgetrennt, über $Na_2SO_4$ getrocknet und eingeengt. Nach säulenchromatographischer Reinigung an Kieselgel (Methyl-tert.-Butylether/n-Hexan) erhielt man 5,4 g (65 % Ausbeute) der Titelverbindung als farblose Kristalle vom Schmelzpunkt 119 bis 121 °C.

$^1$H-NMR ($CDCl_3$): δ = 2,17 (s, 3H); 2,86 (d, 3H); 3,94 (s, 3H); 5,11 (s, 2H); 6,71 (sbr, 1H); 7,19 - 7,55 (m, 8H) ppm.

Beispiel 14

S-Methyl-(Z)-2-methoxyimino-2-{2'-(E)-1''-[2-naphthyl]-1''-methyliminooxymethyl}-phenylthioacetimidat ($R^1 = CH_3$, $R^2 = CH_3$, $R^3{}_m = 3,4\text{-}CH=CH\text{-}CH=CH$) wurde entsprechend hergestellt und hat einen Schmelzpunkt von 106 bis 108 °C.

Anwendungsbeispiele

1. Die insektizide Wirkung der Verbindungen der allgemeinen Formel I ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt. Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimal- konzentration).

2. Die fungizide Wirkung der Verbindungen der allgemeinen Formel I ließ sich durch folgende Versuche zeigen:

Die Wirkstoffe wurden als 20 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

Nach Abschluß der Versuche wurde der Befall der behandelten Pflanzen im Verhältnis zu den unbehandelten Pflanzen beurteilt.

Erysiphe graminis var. tritici (Weizenmehltau)

Blätter von Weizenkeimlingen (Sorte "Frühgold") wurden zunächst mit der wäßrigen Aufbereitung der Wirkstoffe behandelt. Nach ca. 24 h wurden die Pflanzen mit Sporen des Weizenmehltaus (Erysiphe graminis var. tritici) bestäubt. Die so behandelten Pflanzen wurden anschließend für 7 Tage bei 20 bis 22 °C und einer relativen Luftfeuchtigkeit von 75 - 80 % inkubiert. Anschließend wurde das Ausmaß der Pilzentwicklung ermittelt.

In diesem Test zeigten die mit 63 ppm Aufbereitungen der Verbindungen Beispiel Nr. 5 und Nr. 7 behandelten Pflanzen einen Befall von 15 % und weniger, während die unbehandelten Pflanzen zu 65 % befallen waren.

## Patentansprüche

**1.** Thioimidate der Formel I

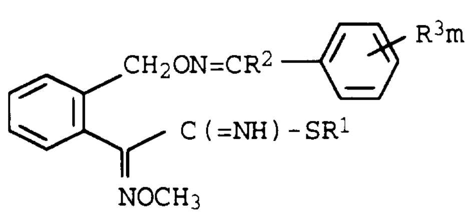

I

in der die Substituenten und der Index die folgende Bedeutung haben:

| | |
|---|---|
| $R^1$ | $C_1$-$C_6$-Alkyl; |
| $R^2$ | $C_1$-$C_3$-Alkyl oder Cyclopropyl; |
| m | 0, 1, 2 oder 3, wobei die Reste $R^3$ verschieden sein können, wenn m für 2 oder 3 steht; |
| $R^3$ | Cyano, Nitro, Hydroxy, Mercapto, Halogen, Amino, $C_1$-$C_6$-Alkyl, Cyano-$C_1$-$C_6$-alkyl, Nitro-$C_1$-$C_6$-alkyl, $C_1$-$C_6$-Alkoxy-$C_1$-$C_6$-alkyl, $C_1$-$C_6$-Halogenalkyl, $C_3$-$C_6$-Cycloalkyl, $C_1$- |

$C_6$-Alkoxy, $C_1$-$C_6$-Halogenalkoxy, $C_3$-$C_6$-Cycloalkoxy, $C_1$-$C_6$-Alkylthio, $C_1$-$C_6$-Alkylamino, Di-$C_1$-$C_6$-alkylamino,
$C_2$-$C_6$-Alkenyl, $C_2$-$C_6$-Alkenyloxy, $C_2$-$C_6$-Alkinyl, $C_2$-$C_6$-Alkinyloxy,
Phenyl, Phenoxy,
$C(=O)R^a$, $C(=O)OR^a$, $C(=O)NR^aR^b$, $C(=S)NR^aR^b$, $OC(=O)R^a$, $NR^aC(=O)R^b$, $NR^aC(=O)OR^b$,
$S(=O)R^a$, $S(=O)OR^a$,
$CR^a=NR^b$, $C(R^a)=NOR^b$, $C(XR^a)=NOR^b$, $N=CR^aR^b$, oder zwei benachbarte $R^3$ zusammen eine Gruppe $CH=CH-CH=CH$;

X Sauerstoff, Schwefel oder Amino ($NR^c$);
$R^a$,$R^b$,$R^c$ unabhängig voneinander Wasserstoff oder $C_1$-$C_6$-Alkyl,

sowie deren Salze.

2. Verfahren zur Herstellung der Verbindungen I gemäß Anspruch 1, dadurch gekennzeichnet, daß man ein Toluolderivat der Formel II

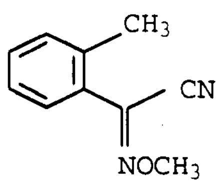

II

in an sich bekannter Weise mit einem Halogenierungsmittel in das entsprechende Benzylhalogenid der Formel III

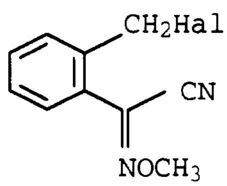

III

in der Hal für ein Halogen steht, überführt, III anschließend mit einem Oxim der Formel IV

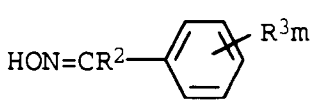

IV

zum entsprechenden Oximether V

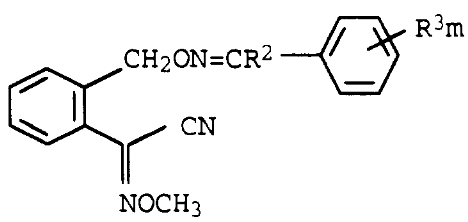

V

verethert, V danach mit Schwefelwasserstoff in Gegenwart einer Base in ein Thiocarbonsäureamid VI

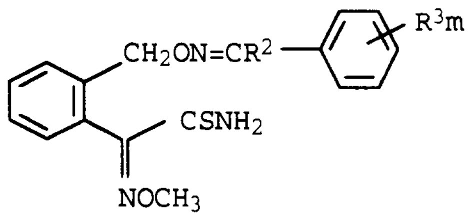

VI

überführt und VI mit einem Alkylierungsmittel zu I umsetzt.

**3.** Verfahren nach Anspruch 2, dadurch gekennzeichnet, daß man den Oximether der Formel V gemäß Anspruch 2 zu einer Carbonsäure der Formel VII

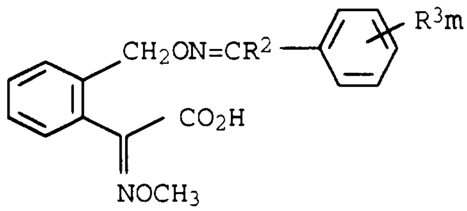

VII

hydrolysiert, VII anschließend mit Ammoniak in das entsprechende Carbonsäureamid VIII

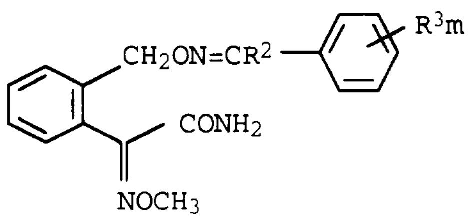

VIII

überführt und VIII mit zunächst einem Schwefelreagenz und anschließend mit einem Alkylierungsmittel zu I umsetzt.

**4.** Verfahren nach den Ansprüchen 2 und 3, dadurch gekennzeichnet, daß man von einem Toluolderivat der Formel II gemäß Anspruch 2 ausgeht, in dem die C=N-Doppelbindung bezüglich der Reste -C≡N und $-OCH_3$ Z-konfiguriert ist.

**5.** Zwischenprodukte der allgemeinen Formel IX

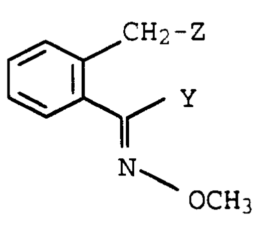

IX

in der die Substituenten die folgende Bedeutung haben:

Y CN, $C(=S)NH_2$, $CO_2H$ oder $CONH_2$, und

Z Wasserstoff, Halogen oder eine Gruppe

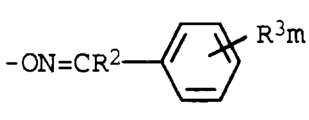

wobei $R^2$, $R^3$ und m die in Anspruch 1 gegebene Bedeutung haben.

**6.** Verwendung der Verbindungen der Formel IX als Zwischenprodukt.

**7.** Mittel gegen tierische Schädlinge oder Schadpilze, enthaltend übliche Zusatzstoffe und eine wirksame Menge einer Verbindung der Formel I gemäß Anspruch 1.

**8.** Mittel nach Anspruch 7 zur Bekämpfung tierischer Schädlinge aus der Klasse der Insekten, Spinntiere oder Nematoden.

**9.** Verfahren zur Bekämpfung von tierischen Schädlingen oder Schadpilzen, dadurch gekennzeichnet, daß man die Schädlinge oder Schadpilze, deren Lebensraum oder die von ihnen freizuhaltenden Pflanzen, Flächen, Materialien oder Räume mit einer wirksamen Menge einer Verbindung der Formel I gemäß Anspruch 1 behandelt.

**10.** Verwendung der Verbindungen I gemäß Anspruch 1 zur Herstellung von Mitteln gegen tierische Schädlinge oder Schadpilze.

**11.** Verwendung der Verbindungen I gemäß Anspruch 1 zur Bekämpfung von tierischen Schädlingen oder Schadpilzen.

Europäisches Patentamt

# EUROPÄISCHER RECHERCHENBERICHT

Nummer der Anmeldung
EP 94 11 8383

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,Y | EP-A-0 528 681 (SHIONOGI SEIYAKU)<br>* Seite 2; Ansprüche 1,7,11-14; Beispiele 3-6; Tabellen 4-6 *<br>--- | 1,2,7,10 | C07C327/58<br>C07C255/64<br>C07C327/44<br>A01N37/52 |
| D,Y | EP-A-0 463 488 (BASF)<br>* Seite 3 - Seite 5; Ansprüche 1,3,4,6; Beispiele 3,5; Tabelle II * | 1,2,7,10 | |
| X | * Seite 3 - Seite 5 *<br>--- | 5 | |
| X | WO-A-92 13830 (IMPERIAL CHEMICAL INDUSTRIES)<br>* Seite 1; Tabelle III, Verbindung 346 *<br>--- | 5 | |
| X | EP-A-0 498 188 (BASF)<br>* Seite 16, Zeile 9 - Zeile 15 *<br>--- | 5 | |
| D,P,X | EP-A-0 585 751 (BASF)<br>* Seite 3 - Seite 4; Ansprüche 1,4,11,12; Beispiele; Tabellen 1,2 *<br>--- | 5,6 | |
| P,X | WO-A-94 22844 (ZENECA)<br>* Seite 29 *<br>--- | 5 | **RECHERCHIERTE SACHGEBIETE (Int.Cl.6)**<br>C07C |
| E | WO-A-94 26700 (CIBA-GEIGY)<br>* Seiten 1, 8 - 14; Beispiele H-1 - H-3, H - 6; Tabelle 2; Ansprüche 1, 18, 20 *<br>----- | 1,2,5-7, 9-11 | |

Der vorliegende Recherchenbericht wurde für alle Patentansprüche erstellt

| Recherchenort | Abschlußdatum der Recherche | Prüfer |
|---|---|---|
| DEN HAAG | 21.Februar 1995 | English, R |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)